# EUROPÄISCHE PATENTANMELDUNG

(11) **EP 2 754 425 A1**
(43) Veröffentlichungstag der Anmeldung: **16.07.2014**
(21) Anmeldenummer: 13196825.7
(22) Anmeldetag: 12.12.2013
(51) Int. Cl.: A61F 2/95

(54) **Vorrichtung und Verfahren zum Crimpen eines Implantats**

(30) Priorität: 09.01.2013 US 201361750361 P
(71) Anmelder: Biotronik AG, 8180 Bülach (CH)
(72) Erfinder: Fargahi, Amir, 8180 Bülach (CH)
(74) Vertreter: Galander, Marcus

(57) **Zusammenfassung**

Die vorliegende Erfindung betrifft eine Vorrichtung (10, 10') zum Crimpen eines Implantats (20), insbesondere einer intraluminalen Endoprothese, welches zumindest über einen Teil seiner Länge entweder einen komprimierten Zustand oder einen expandierten Zustand einnehmen kann. Eine einfach handhabbare, kleinbauende und kostengünstige Crimpvorrichtung wird erfindungsgemäß dadurch realisiert, dass ein hohlzylinderund/oder hohlkegelförmiges, vorzugsweise geflochtenes Drahtnetz vorgesehen ist, in dessen Innenvolumen (14, 14') das Implantat (20) im expandierten Zustand zumindest mit einem Abschnitt einlegbar ist, wobei das Drahtnetz durch Aufbringen einer Zugkraft an mindestens einem Ende in Längsrichtung und/oder durch Aufbringen einer Druckkraft in radialer Richtung gleichzeitig sowohl eine Verlängerung als auch eine Verkleinerung des Innendurchmessers (d, d') derart erfährt, dass das Implantat (20) hierdurch wenigstens entlang eines Abschnitts von dem expandierten Zustand in den komprimierten Zustand überführbar ist. Die Erfindung betrifft ferner ein entsprechendes Verfahren zum Crimpen eines Implantats.

## Beschreibung

Die vorliegende Erfindung betrifft eine Vorrichtung zum Crimpen eines Implantats, insbesondere einer intraluminalen Endoprothese, und ein entsprechendes Verfahren.

Medizinische Implantate, insbesondere intraluminale Endoprothesen, für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents (Gefäß-Stents (vaskuläre Stents, einschließlich Stents für die Anwendung im Bereich des Herzens und Herzklappenstents, z.B. Mitralklappen-Stent, Pulmonalklappenstent) und Gallengang-Stents), Endoprothesen zum Verschließen von persistierenden Foramen ovale (PFO), Stentgrafts zur Behandlung von Aneurysmen, Endoprothesen zum Verschließen eines ASD (Vorhofscheidewanddefekt, atrial septal defect) sowie Prothesen im Bereich des Hart- und Weichgewebes zu verstehen. Ein derartiges Implantat wird häufig mittels eines Katheters in das zu behandelnde Organ oder Gefäß eingesetzt.

Stents und andere Implantate weisen in vielen Fällen eine durchbrochene hohlzylinderförmige (rohrförmige) und/oder hohlkegelförmige Grundstruktur auf, die an beiden Längsenden offen ist, wobei die Grundstruktur häufig aus einer Vielzahl von Stegen zusammengesetzt ist. In einer derartige Grundstruktur können z.B. bei einem Herzklappenstent auf der Innenseite Klappensegel, beispielsweise drei Klappensegel, angeordnet sein, welche die Herzklappe ausbilden und aus einem Kunststoff oder einem biologischen Material, z.B. Schweine-Pericard, bestehen können. In diesem Fall trägt der Stent die Herzklappe und verankert diese im Herzen.

Stents und andere Implantate nehmen üblicherweise zwei Zustände ein, nämlich einen komprimierten Zustand mit einem kleinen Durchmesser und einen expandierten Zustand mit einem größeren Durchmesser. Im komprimierten Zustand kann das Implantat mittels eines Katheters in das zu behandelnde Gefäß oder Organ durch enge Gefäße hindurch eingeführt und an der zu behandelnden Stelle positioniert werden. Hierfür wird das Implantat gecrimpt und dadurch wenigstens über einen Teil seiner Länge von dem expandierten Zustand mit größerem Durchmesser in den komprimierten Zustand mit kleinerem Durchmesser überführt. Am Ort der Behandlung wird das Implantat dann beispielsweise mittels des Ballons des Katheters dilatiert und nimmt dann den wieder den expandierten Zustand ein, in dem das Implantat in dem Gefäß oder Organ verbleibt und dort festgelegt ist, nachdem der Katheter aus dem Körper des Behandelten entfernt wurde. Alternativ nimmt ein Implantat in dem Fall, in dem seine Grundstruktur aus einem selbstexpandierenden Material (z.B. Nitinol) besteht, den komprimierten Zustand durch Komprimierung unterhalb der Übergangstemperatur und den expandierten Zustand oberhalb der Übergangstemperatur ein.

Aus der Druckschrift US 8,029,564 B2 ist eine Herzklappenprothese und eine Umlenkeinrichtung bekannt. Das System beinhaltet ferner ein Band, das durch die freien Enden der Stentstangen, welche die Herzklappe tragen, hindurch geführt wird. Mittels dieses Bandes können die Stentstangen nach innen umgebogen werden, um einen geschlossenen Zustand zu erreichen. Dieses System eignet sich jedoch nicht dafür, nichtinvasiv mittels eines Katheters zu der behandelnden Stelle transferiert zu werden, da es im Fußbereich zu voluminös ist.

Aus der Druckschrift US 2011/0056064 A1 ist ein Crimpwerkzeug bekannt, das sehr kostenintensiv in der Herstellung ist, da es einen komplexen Aufbau mit einer Vielzahl von präzise zu fertigenden Teilen aufweist, welche sich synchron bewegen müssen. Das Crimpwerkzeug setzt sich insbesondere aus einer Vielzahl von Riegeln zusammen, die entlang eines Kreisumfangs nebeneinander und um eine quer zum jeweiligen Riegel verlaufende Achse drehbar angeordnet sind. In die zwischen den Enden der Riegel ausgebildete Öffnung ist das zu crimpende Implantat aufnehmbar. Durch die Bewegung eines Hebels werden Führungpins, wobei jeweils ein Pin an einem vorderen Ende eines Riegels angeordnet ist, in einem Langloch derart verschoben, dass der Radius der zwischen den Riegeln ausgebildeten Öffnung verringert oder vergrößert wird. Für das Crimpen eines Herzklappenstents werden die Pins derart verschoben, dass der Radius der Öffnung verkleinert wird. Bei diesem Werkzeug ist der Stent während des Crimpvorgangs vollständig durch dieses überdeckt, da die Riegel und die Pins zwischen zwei Platten angeordnet sind und das Werkzeug daher in Längsrichtung des Stents eine große Ausdehnung und ein vergleichsweise hohes Gewicht aufweist. Aus diesem Grund kann das Implantat während des Crimpens visuell nicht überwacht werden. Ferner ist nicht auszuschließen, dass die Riegel bei der Verkleinerung des Öffnungs-Radius' überlappen, so dass das Risiko einer Verletzung des Implantats sowie der an dem Implantat angeordneten Gewebeteile hoch ist. Die große Masse des Werkzeugs führt ferner dazu, dass dieses nur mit großem Aufwand unter die Übergangstemperatur bringbar ist, was notwendig ist, wenn das Implantat aus einem selbst expandierenden Material besteht.

Die Druckschrift EP 2 229 921 A1 offenbart eine Vorrichtung zum Crimpen, welche eine Vielzahl von entlang einer Zylinder-Mantellinie zwischen zwei beabstandeten Ringen gespannten Drähten aufweist. Wird einer dieser Ringe relativ zu dem zweiten Ring um eine Achse parallel zu diesen Mantellinien gedreht, so werden die Drähte zu der gemeinsamen Achse verdreht, so dass diese eine Uhrglas-ähnliche geometrische Fläche ähnlich zu einem Rotationshyperboloiden ausbilden. Die durch den Abschnitt mit dem kleinsten Durchmesser definierte Öffnung, welche diese Drähte zwischen sich ausbilden, wird bei zunehmender Verdrehung der Ringe verkleinert oder vergrößert. In diese Öffnung kann ein Implantat angeordnet und bei Verkleinerung der Öffnung gecrimpt werden. Diese bekannte Vorrichtung ist ebenfalls vergleichsweise kompliziert aufgebaut, so dass deren Montage schwierig ist. Das Implantat wird während des Crimpens durch die Drähte der Vorrichtung überdeckt, so dass der Crimpprozesses auch bei dieser bekannten Vorrichtung nicht visuell überwacht werden kann. Nachteilhaft ist ferner, dass die Drähte an den beiden Enden, an denen die Drähte an den gegenüber liegenden Ringen befestigt sind, stärker gespannt werden als in ihrem mittleren Bereich. Dies führt zu einer ungleichmäßigen Verteilung der Kompressionskraft auf das Implantat. Aufgrund der vielen Drähte und der notwendigen großen Ringe an deren Enden ist die bekannte Vorrichtung vergleichsweise groß und schwer.

Die Aufgabe besteht somit darin, eine Vorrichtung zum Crimpen eines derartigen Implantats zu schaffen, welche weniger kompliziert aufgebaut sowie kleiner und handlicher gebaut ist. Entsprechend soll ein Verfahren zum Crimpen angegeben werden, welches zuverlässig und einfach durchführbar ist.

Die obige Aufgabe wird durch eine Vorrichtung mit den Merkmalen des Anspruchs 1 gelöst.

Insbesondere weist die erfindungsgemäße Vorrichtung ein hohlzylinder- und/oder hohlkegelförmiges geflochtenes Drahtnetz auf, in dessen Innenvolumen das Implantat im expandierten Zustand zumindest mit einem Abschnitt oder vollständig einlegbar ist, wobei das Drahtnetz durch Aufbringen einer Zugkraft an mindestens einem Ende in Richtung der Längsachse und/oder durch Aufbringen einer Druckkraft in radialer Richtung gleichzeitig sowohl eine Verlängerung als auch eine Verkleinerung des Innendurchmessers derart erfährt, dass das in das Innenvolumen des Drahtnetzes eingelegte Implantat wenigstens entlang eines Abschnitts (d.h. entlang eines Teils seiner Länge) oder entlang seiner gesamten Länge durch die Verkleinerung des Innendurchmessers des Drahtnetzes von dem expandierten Zustand in den komprimierten Zustand überführbar ist. Das Drahtnetz wird im Folgenden auch als Braid bezeichnet. Durch die erfindungsgemäße Vorrichtung kann beispielsweise eine Reduktion des Innendurchmessers auf höchstens 1/4 des Innendurchmessers im expandierten Zustand, vorzugsweise auf höchstens 1/5 des Innendurchmessers im expandierten Zustand, erreicht werden.

Die oben angegebene Vorrichtung ist vergleichsweise kleinbauend aufgebaut. Der Innendurchmesser des Drahtnetzes ist im Ausgangszustand, in dem das Implantat im expandierten Zustand in das Innenvolumen einlegbar ist, lediglich geringfügig größer als der Außendurchmesser des Implantats. Auch die Länge des Drahtnetzes in Längsrichtung ist vorzugsweise geringfügig größer als die Länge des Implantats oder die Länge des zu crimpenden Abschnitts des Implantats.

Die Ausbildung der erfindungsgemäßen Vorrichtung als Drahtnetz (Braid) bedingt einen einfachen und leichten Aufbau der erfindungsgemäßen Vorrichtung. Beispielsweise besteht das Drahtnetz aus einem Polymer oder einer Metall-Legierung, wobei die Metall-Legierung vorzugsweise mindestens eines der Elemente der Gruppe Eisen (Fe), Kobalt (Co), Chrom (Cr), Nickel (Ni) enthält. Besonders bevorzugt besteht das Drahtnetz aus Edelstahl oder einer Cobaltbasis-Superlegierung. Das Braid ist elastisch, homogen und gleichmäßig verformbar, so dass es sich ausdehnen und in alle Richtungen biegen kann. Das Braid besitzt besonders bevorzugt selbst expandierende Eigenschaften, insbesondere kann das Braid aus einer Phynox (Elgiloy) - Legierung bestehen. Phynox (Elgiloy) ist eine austenitische härtbare Superlegierung auf Cobaltbasis (40 Gew.% Co, 20 Gew.% Cr, 16 Gew.% Ni und 7 Gew.% Mo). Ihre mechanische Festigkeit kann nach dem 2.600 N/mm² übersteigen. Die maximale Zugfestigkeit hängt stark von der zuvor durchgeführten Kaltbearbeitung ab. Die Legierung ist nichtmagnetisch, sehr korrosionsbeständig (korrosionsbeständiger als jeder andere Edelstahl) und temperaturbeständig. Ferner besitzt die Legierung einen hohen Elastizitätsmodul (220 kN/mm²) kombiniert mit einer Dehnungsgrenze von 1.800 N/mm², so dass die Legierung ausgesprochen gut für das erfindungsgemäße Braid geeignet ist. Die Legierung wird von der Fa. Lamineries MATTHEY SA angeboten.

Wie oben bereits ausgeführt, wird durch Langziehen des Braids eine Innendurchmesser-Reduktion bewirkt. Im umgekehrten Fall führt das Ausdehnen des Drahtnetzes derart, dass der Innen- und Außendurchmesser vergrößert wird, zu einer Verkürzung des Drahtnetzes. Die für das Braid einsetzbaren Metall-Legierungen sind vorzugsweise keine Formgedächtnis-Legierungen, so dass insbesondere im Temperaturbereich von 0°C bis 40°C keine Phasenumwandlung vorliegt. In diesem Temperaturbereich kann daher ohne Phasenumwandlungs-Einflüsse eine kontinuierliche Verlängerung des Drahtnetzes mit Durchmesser-Reduktion bzw. eine Verkürzung mit Durchmesser-Vergrößerung reversibel erfolgen.

In einem weiteren Ausführungsbeispiel weist das Drahtnetz einen ersten hohlzylinderförmigen und einen zweiten hohlkegelförmigen Abschnitt auf. Bei diesem Ausführungsbeispiel wird das Implantat im hohlzylinderförmigen Abschnitt eingeführt und in vorteilhafter Weise im hohlkegelförmigen Abschnitt gecrimpt bzw. komprimiert. Diese Form begünstigt das Crimpen.

Um eine besonders einfache Durchmesser-Reduktion zu erreichen, ist an mindestens einem ersten Ende in Längsrichtung (Richtung der Längsachse) des Drahtnetzes ein Ring vorgesehen, welcher gegenüber dem zweiten Ende des Drahtnetzes in Richtung der Längsachse, vorzugsweise entlang einer Schiene, verschieblich ist. Dabei wird das zweite Ende des Drahtnetzes örtlich fest gehalten. Durch das Verschieben des Rings wird eine Zugkraft auf das Drahtnetz aufgebracht. Hierfür ist der Ring an dem jeweiligen Ende des Drahtnetzes befestigt. In einem bevorzugten Ausführungsbeispiel weist das Braid an beiden Enden in Längsrichtung einen Ring auf. Die Schiene erstreckt sich vorzugsweise parallel zur Längsachse des hohlzylinder- oder hohlkegelförmigen Drahtnetzes.

Da die Vorrichtung, wie oben beschrieben, kleinbauend und leicht ausgebildet ist, kann diese leicht in ein Kühlmittel eingebracht werden, so dass das Implantat und das Drahtnetz auf eine Temperatur unterhalb der Übergangstemperatur abkühlbar ist. Als ein derartiges Kühlmittel kann beispielsweise 0°C kaltes Wasser verwendet werden.

Die obige Aufgabe wird ferner durch ein einfaches Verfahren zum Crimpen eines Implantats mit den Merkmalen des Anspruchs 5 gelöst.

Hierbei werden insbesondere die folgenden Schritte durchgeführt. In einem ersten Schritt wird das zunächst im expandierten Zustand vorliegende Implantat zumindest mit einem Abschnitt oder vollständig in das Innenvolumen eines hohlzylinder- oder hohlkegelförmigen, vorzugsweise geflochtenen Drahtnetzes eingelegt. Danach wird durch Aufbringen einer Zugkraft an mindestens einem Ende des Drahtnetzes in Richtung dessen Längsachse und/oder durch Aufbringen einer Druckkraft in radialer Richtung auf das Drahtnetz derart der Innendurchmesser des Drahtnetzes verkleinert, dass das eingelegte Implantat wenigstens entlang eines Abschnitts von dem expandierten Zustand in den komprimierten Zustand überführt wird. Dieses Verfahren ist sehr einfach und kostengünstig durchführbar. Es kann entweder per Hand oder auch in einem automatisierten Prozess realisiert werden.

In einem weiteren Ausführungsbeispiel werden das Implantat und vorzugsweise auch die Vorrichtung vor Überführung des Implantats in den komprimierten Zustand in einem Kühlmittel unter die Übergangstemperatur abgekühlt.

Auf einfache Weise kann eine Zugkraft in Längsrichtung des Drahtnetzes auf dieses dadurch aufgebracht werden, dass ein an einem ersten Ende des Drahtnetzes in Längsrichtung angebrachter Ring vorzugsweise auf einer Schiene relativ zu dem feststehenden, gegenüber liegenden zweiten Ende des Drahtnetzes verschoben wird, wobei die Schiene vorzugsweise parallel zur Längsrichtung verläuft. Als Längsrichtung des Drahtnetzes bzw. des Implantats wird in der vorliegenden Beschreibung der Erfindung die Richtung bezeichnet, die parallel zur Längsachse des Drahtnetzes bzw. des Implantats verläuft.

Nach dem Überführen in den komprimierten Zustand kann in einem weiteren Ausführungsbeispiel der entsprechende Abschnitt des Implantats in einen rohrförmigen Außenschaft eines Katheters eingeführt werden, um den komprimierten Zustand gegebenenfalls auch oberhalb der Übergangstemperatur zu fixieren bzw. das Einführen des Implantats in den zu behandelnden Körper zu realisieren.

In einer Weiterbildung der Erfindung wird danach ein zweiter Abschnitt des Implantats, der zunächst nicht in den Außenschaft eingeführt wurde, vorzugsweise mittels der oben beschriebenen erfindungsgemäßen Vorrichtung oder des oben beschriebenen erfindungsgemäßen Verfahrens ebenfalls in den komprimierten Zustand überführt und anschließend ebenfalls in den Außenschaft eingeführt.

Weitere Ziele, Merkmale, Vorteile und Anwendungsmöglichkeiten der Erfindung ergeben sich auch aus der nachfolgenden Beschreibung von Ausführungsbeispielen zu der Erfindung anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch:
- Fig. 1a: ein erstes Ausführungsbeispiel einer erfindungsgemäßen Vorrichtung in einer Ansicht von der Seite im Ausgangszustand,
- Fig. 1b: ein Kreuzungspunkt einer erfindungsgemäßen Vorrichtung in einer Ansicht von der Seite im expandierten Zustand,
- Fig. 1c: eine Masche einer erfindungsgemäßen Vorrichtung in einer Ansicht von der Seite,
- Fig. 1d: die Masche von Fig. 1c in einer Ansicht von der Seite im expandierten Zustand und im komprimierten Zustand,
- Fig. 2: das zu crimpende Implantat in einer Ansicht von der Seite,
- Fig. 3: ein Behälter mit Kühlmittel, in dem das zu crimpende Implantat gekühlt wird, in einer perspektivischen Ansicht von der Seite,
- Fig. 4: einen Abschnitt der erfindungsgemäßen Vorrichtung gemäß Fig. 1 in einer Ansicht von der Seite mit darin angeordnetem Implantat im expandierten Zustand,
- Fig. 5: den Abschnitt gemäß Fig. 4 in einem ersten Zwischenschritt des Crimpprozesses in einer Ansicht von der Seite,
- Fig. 6: den Abschnitt gemäß Fig. 4 in einem zweiten Zwischenschritt des Crimpprozesses in einer Ansicht von der Seite,
- Fig. 7: das Implantat nach dem Crimpen im komprimierten Zustand in einer Ansicht von der Seite, wobei das Drahtnetz in den Ausgangzustand zurück verformt wurde,
- Fig. 8: das Einführen des gecrimpten Implantats in einen Außenschaft eines Katheters in einer Ansicht von der Seite,
- Fig. 9 und 10: das in den Außenschaft eines Katheters teilweise eingeführte Implantat angeordnet in der erfindungsgemäßen Vorrichtung gemäß Fig. 1, wobei in Fig. 10 das Implantat nach einem weiteren Crimpen vollständig, d.h. über seine gesamte Länge, in dem Außenschaft angeordnet ist, in einer Ansicht von der Seite,
- Fig. 11: das vollständig in dem Außenschaft eines Katheters angeordnete Implantat nach Abschluss des Crimpprozesses in einer Ansicht von der Seite,
- Fig. 12: ein zweites Ausführungsbeispiel einer erfindungsgemäßen Vorrichtung in einer perspektivischen Ansicht von der Seite im Ausgangszustand und
- Fig. 13: das Ausführungsbeispiel gemäß Fig. 12 in einer perspektivischen Ansicht von der Seite im Endzustand nach Abschluss des Crimpprozesses.

Die Figuren zeigen zwei Ausführungsbeispiele einer erfindungsgemäßen Vorrichtung schematisch und vereinfacht und stellen insbesondere die Details dar, die wichtig sind, um die Erfindung zu verstehen. Für die Erfindung unbedeutende Details wurden teilweise weggelassen. Weiterhin bedeutet die Bezeichnung "distales Ende" im Zusammenhang mit der vorliegenden Erfindung das Ende des Implantats, das während dem Einbringen des Implantats in den Körper von dem behandelnden Arzt weg zeigt, während das "proximale Ende" zu der z.B. einen Katheter bedienenden Person hin zeigt.

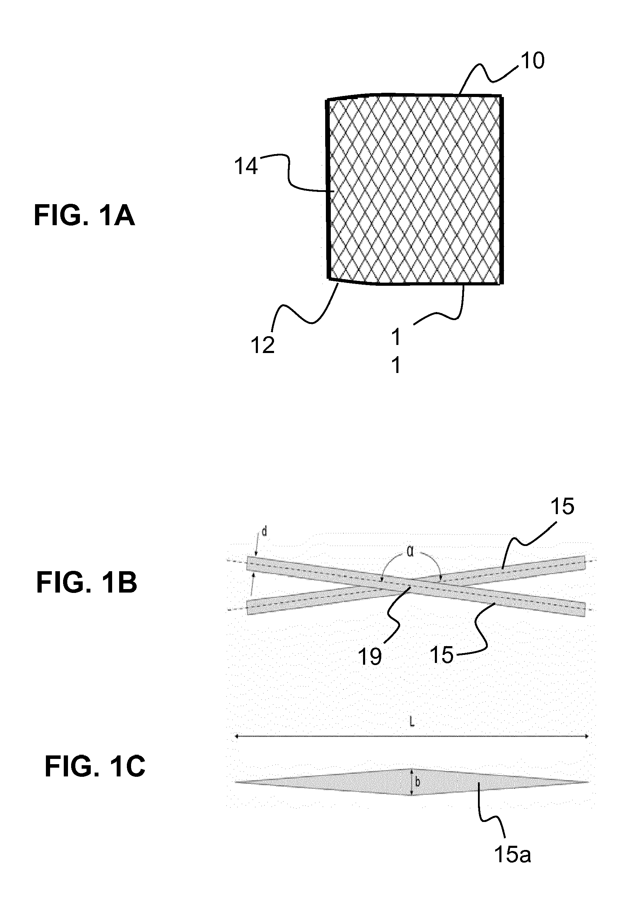

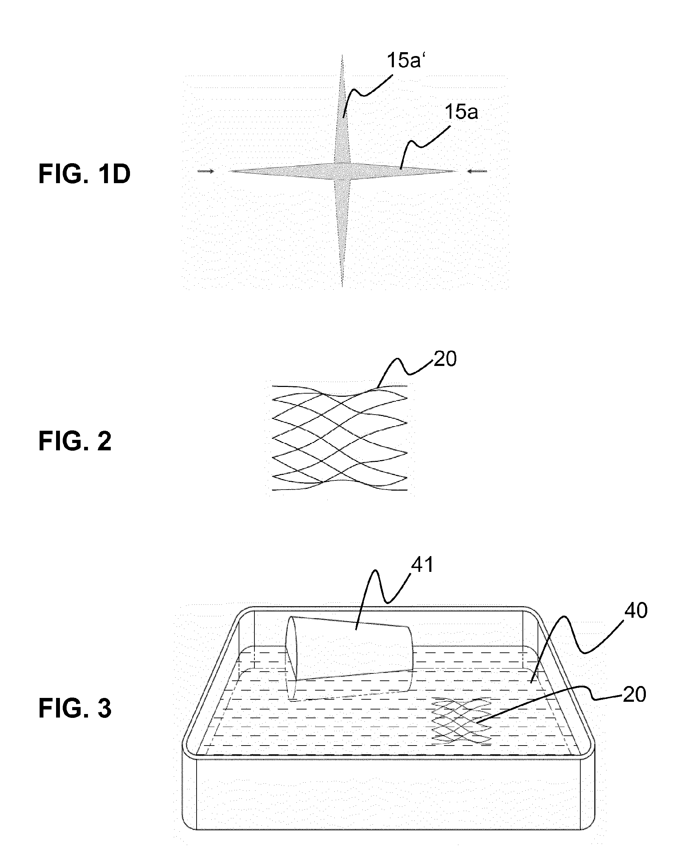

Das in den Fig. 1 bis 7 sowie 9 bis 10 dargestellte Ausführungsbeispiel einer Vorrichtung 10 zum Crimpen eines Implantats 20 (z.B. Herzklappenstent) weist in einem ersten Abschnitt 11 ein hohlzylinderförmiges und in einem zweiten Abschnitt 12 ein hohlkegelförmiges geflochtenes Drahtnetz auf.

Hierbei kann das Drahtnetz beispielsweise aus Drähten 15 mit kreisförmigen Querschnitt bestehend aus einer Phynox (Elgiloy) Legierung (Co-Cr-Ni-Legierung, siehe oben) zusammengesetzt sein, welche einen Drahtdurchmesser d von 0,2 mm aufweisen. Der Winkel zwischen den Drähten in den Kreuzungspunkten beträgt im expandierten Zustand z.B. α = 175°. Die Maschenlänge L der durch die Drähte 15 aufgespannten Masche 15a beträgt 5 mm und die Maschenbreite b beträgt 0,9 mm im expandierten Zustand. Über den gesamten Umfang des Drahtnetzes sind fünfzehn Kreuzungspunkte 19 angeordnet. Ein Kreuzungspunkt 19 eines derartigen Drahtnetzes ist in Fig. 1b dargestellt. Eine durch Drähte 15 aufgespannte Masche 15a ist im expandierten Zustand in den Fig. 1c und 1d gezeigt. Durch das Auseinanderziehen des Drahtnetzes wird die Masche in den in Fig. 1d gezeigten Zustand 15a' überführt. Die auf die Masche 15a während der Komprimierung einwirkenden Kräfte sind durch die außen liegenden Pfeile in Fig. 1d veranschaulicht. Das in den Fig. 1a bis 1d gezeigte Ausführungsbeispiel einer erfindungsgemäßen Vorrichtung besteht, wie oben ausgeführt, aus Runddraht, kann aber gleichwohl aus Flachdraht oder einer Kombination beider Drahttypen zusammengesetzt sein.

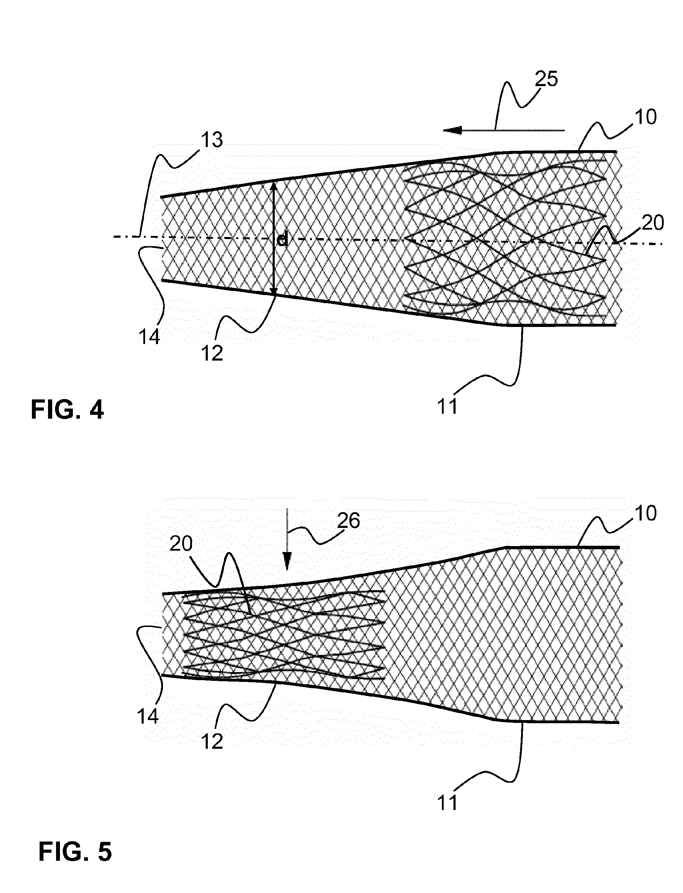

In dem in Fig. 1a gezeigten Ausgangszustand vor dem Crimpen kann das Implantat 20, wie in Fig. 4 gezeigt, im expandierten Zustand in das Innenvolumen 14 des Drahtnetzes eingeführt werden. Das Innenvolumen 14 wird durch den von dem Hohlkegel bzw. Hohlzylinder umschlossenen Hohlraum gebildet. Die Richtung, die in Fig. 4 mit dem Pfeil 25 veranschaulicht ist, erstreckt sich dabei parallel zur Längsachse 13 des Drahtnetzes in den Abschnitten 11 und 12. Das Implantat 20 wird vorzugsweise von der Seite eingeführt, auf der das Drahtgitter den Hohlzylinder 11 ausbildet.

In einem bevorzugten Ausführungsbeispiel wurde das Implantat 20 vor dem Einbringen in die Crimpvorrichtung 10, wie in Fig. 3 dargestellt, in einem Kühlmittel 40 auf eine Temperatur unterhalb der Übergangstemperatur, beispielsweise auf 0°C, abgekühlt. Um das Kühlmittel 40, das vorzugsweise Wasser ist, auf der Temperatur von 0°C zu halten, schwimmt in dem Wasserbad ein Eisblock 41. In einem weiteren Ausführungsbeispiel kann der gesamte Crimpvorgang kann in dem Kühlmittel 40 durchgeführt werden.

Nach dem Einlegen des Implantats 20 in das Drahtnetz der Crimpvorrichtung 10 wird im nächsten Schritt, nachdem das Implantat in den hohlkegelförmigen Abschnitt 12 der Crimpvorrichtung 10 vorgeschoben wurde, diese in Längsrichtung (siehe Pfeil 25) auseinandergezogen oder es wird in radialer Richtung, die quer zur Längsrichtung verläuft, wie dies in Fig. 5 mit dem Pfeil 126 veranschaulicht ist, ein Druck von außen aufgebracht, so dass sich der Innendurchmesser d des Innenvolumens 14 verringert und das Implantat 20 gecrimpt wird. Beispielsweise wird durch den äußeren Druck der Innendurchmesser D auf 1/5 des Wertes im expandierten Zustand reduziert.

In der nachfolgenden Tabelle wird das obige Beispiel noch einmal anhand von Zahlen konkretisiert.

| Material der Crimpvorrichtung 10 | z.B. Phynox (Elgiloy) (Co Cr Ni Legierung) |
|---|---|
| Durchmesser d des Drahtes des Drahtnetzes | 0,2 mm |
| Winkel α zwischen den Drähten im Kreuzungspunkt im expandierten Zustand | 175° |
| Maschenlänge L (siehe Fig. 1c) | 5 mm |
| Maschenbreite b (siehe Fig. 1c) | 0,9mm |
| Anzahl der Kreuzungspunkte entlang des Umfangs des Drahtnetzes | 15 |
| Umfang des Drahtnetzes im komprimierten Zustand | 15 x 0,9 mm = 13,5 mm |
| Umfang des Drahtnetzes im expandierten Zustand | 15 x 5 mm = 75 mm |
| Kompressionsverhältnis Umfang (Umfang im expandierten Zustand zu Umfang im komprimierten Zustand) | 455% (75 mm => 13,5 mm) |
| Änderung des Innen-Durchmessers beim Übergang vom expandierten Zustand in den komprimierten Zustand | 21,2 mm (23 mm - 0,8 mm) => 3,4 mm (4,2 mm - 0,8 mm) |

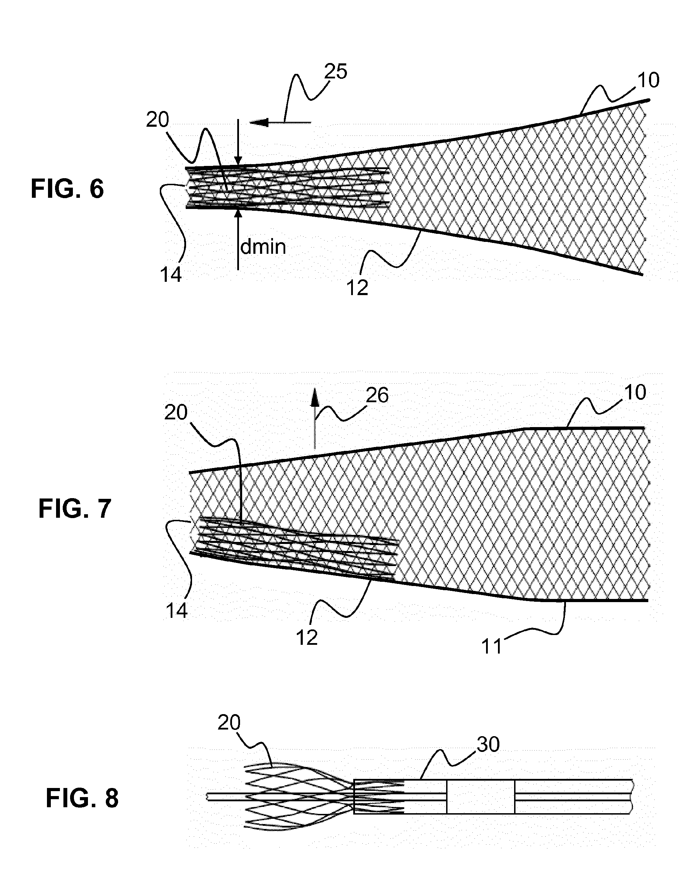

Fig. 6 zeigt das Implantat 20 im komprimierten Zustand, wobei die Crimp-Vorrichtung 10 in diesem Bild ihren Zustand mit dem geringsten Innendurchmesser Dmin zeigt. Nun kann das Crimp-Werkzeug 10 in Längsrichtung wieder verkürzt oder in radialer Richtung (Pfeil 26) auseinander gezogen werden, so dass der Innendurchmesser D zunimmt. Dies ist in Fig. 7 dargestellt. In diesem Zustand kann das Implantat 20 aus der Crimpvorrichtung 10 herausgenommen werden.

Anschließend wird das Implantat 20 mit seinem gecrimpten Abschnitt in einen Außenschaft 30, der beispielsweise als Polymerschlauch ausgebildet ist, eingeführt (vgl. Fig. 8).

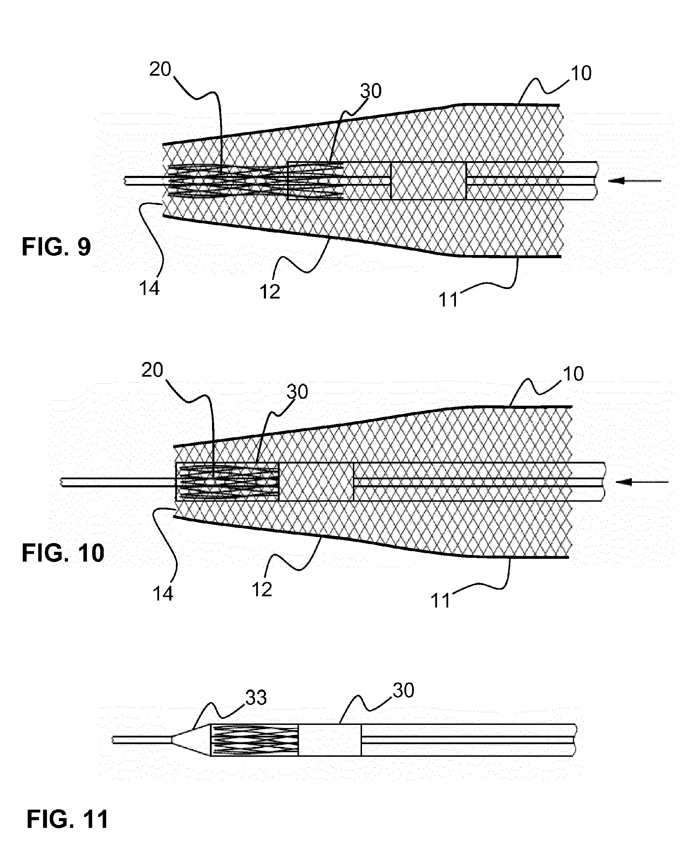

In einem nächsten Schritt, der in Fig. 9 dargestellt ist, wird nun das Implantat 20 zusammen mit dem Außenschaft 30 noch einmal in die Crimpvorrichtung 10 eingeführt, um den Abschnitt des Implantats 20, der noch aus dem Außenschaft 30 herausragt, zu crimpen. Auch in diesem Schritt wird analog zu dem anhand der Fig. 5 und 6 erläuterten Crimpschritt durch Aufbringen einer Zugkraft entlang der Längsrichtung der Crimpvorrichtung und/oder einer Druckkraft in radialer Richtung eine Reduktion des Innendurchmessers der Crimpvorrichtung 10 bewirkt. Im Anschluss daran wird, wie in Fig. 10 dargestellt, der Außenschaft 20 vollständig über das Implantat vorgeschoben, bis diese die Katheterspitze 33 erreicht (vgl. Fig. 11).

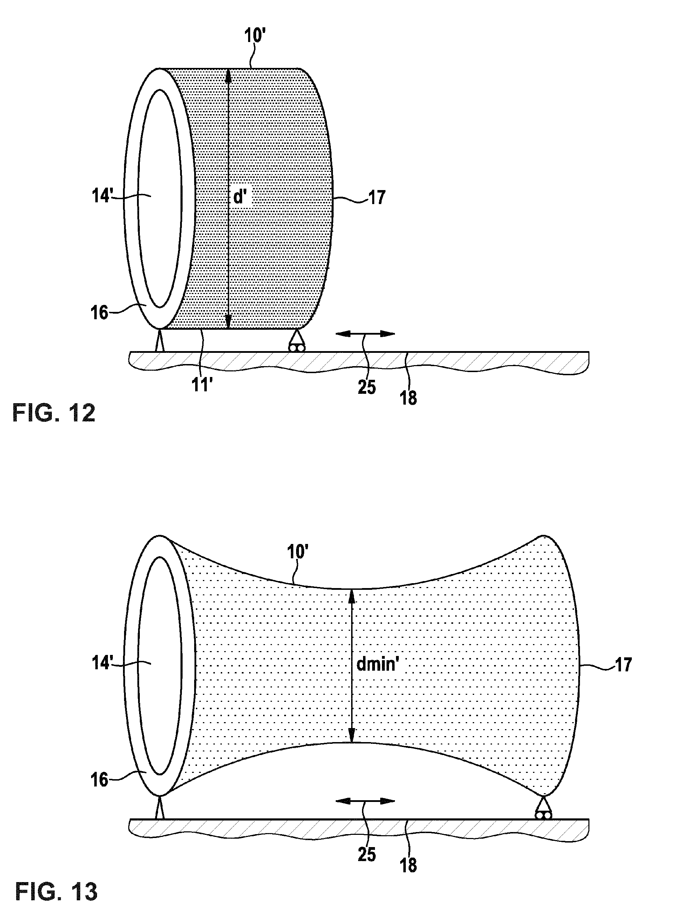

In den Fig. 12 und 13 ist anhand eines weiteren Ausführungsbeispiels einer Crimpvorrichtung dargestellt, wie die Reduktion des Innendurchmessers einfach realisiert werden kann.

Wie in dem in Fig. 1 dargestellten ersten Ausführungsbeispiel weist das zweite Ausführungsbeispiel einer erfindungsgemäßen Vorrichtung 10' ebenfalls ein Drahtnetz auf, welches in diesem Fall hohlzylinderförmig ist. An beiden Enden des Drahtnetzes in Längsrichtung ist jeweils ein Ring 16, 17 vorgesehen, wobei der im Bild auf der linken Seite angeordnete Ring (proximaler Ring) 16 feststeht, während der auf der im Bild rechten Seite angeordnete (distale) Ring 17 verschieblich auf einer Schiene 18 angeordnet ist. Wird nun der distale Ring 17 parallel zur Längsrichtung des Drahtnetzes entlang der Schiene 18 verschoben (Pfeil 25), so wird der Innendurchmesser d' des Innenvolumens 14' insbesondere im mittleren Abschnitt des Drahtnetzes reduziert, bis dort ein minimaler Innendurchmesser dmin' erreicht wird, wenn der distale Ring 17 zum proximalen Ring 16 in Richtung der Schiene 18 den größten Abstand aufweist. Ein in diesem Bereich des Drahtnetzes angeordnetes Implantat (in Fig. 12 und 13 nicht dargestellt) wird durch diese Durchmesserreduktion zuverlässig gecrimpt.

### Bezugszeichenliste

- 10, 10': Crimpvorrichtung
- 11: hohlzylinderförmiger Abschnitt
- 12: hohlkegelförmiger Abschnitt
- 13: Längsachse der Crimpvorrichtung 10
- 14, 14': Innenvolumen
- 15: Draht
- 15a: Masche, durch Drähte 15 aufgespannt im Ausgangszustand
- 15a': Masche 15a nach der Verkleinerung des Innendurchmessers
- 16: proximaler Ring
- 17: distaler Ring
- 18: Schiene
- 19: Kreuzungspunkt
- 20: Implantat
- 25, 26: Pfeil
- 30: Außenschaft
- 33: Katheterspitze
- 40: Kühlmittel
- 41: Eisblock
- b: Maschenbreite
- d: Drahtdurchmesser
- D, D': Innendurchmesser
- Dmin, Dmin': kleinster Innendurchmesser des Drahtnetzes des Implantats 10, 10'
- L: Maschenlänge
- α: Winkel am Kreuzungspunkt der Drähte 15

## Patentansprüche

1. Vorrichtung (10, 10') zum Crimpen eines Implantats (20), insbesondere einer intraluminalen Endoprothese, welches zumindest über einen Teil seiner Länge entweder einen komprimierten Zustand oder einen expandierten Zustand einnehmen kann, **gekennzeichnet durch** ein hohlzylinder- und/oder hohlkegelförmiges, vorzugsweise geflochtenes Drahtnetz, in dessen Innenvolumen (14, 14') das Implantat (20) im expandierten Zustand zumindest mit einem Abschnitt einlegbar ist, wobei das Drahtnetz **durch** Aufbringen einer Zugkraft an mindestens einem Ende in Längsrichtung und/oder **durch** Aufbringen einer Druckkraft in radialer Richtung gleichzeitig sowohl eine Verlängerung als auch eine Verkleinerung des Innendurchmessers (d, d') derart erfährt, dass das Implantat (20) hierdurch wenigstens entlang eines Abschnitts von dem expandierten Zustand in den komprimierten Zustand überführbar ist.

2. Vorrichtung nach Anspruch 1, **dadurch gekennzeichnet, dass** das Drahtnetz aus einem Polymer oder einer Metall-Legierung, die Metall-Legierung vorzugsweise enthaltend mindestens eines der Elemente der Gruppe Fe, Co, Cr und Ni, vorzugsweise aus einem Edelstahl oder einer Cobaltbasis-Superlegierung, besteht.

3. Vorrichtung nach einem der vorhergehenden Ansprüche, **dadurch gekennzeichnet, dass** das Drahtnetz an mindestens einem Ende in Richtung der Längsachse einen Ring (17) aufweist, welcher gegenüber dem anderen Ende des Drahtnetzes in Längsrichtung, vorzugsweise entlang einer Schiene (18), verschieblich ist.

4. Vorrichtung nach einem der vorhergehenden Ansprüche, **dadurch gekennzeichnet, dass** die Vorrichtung in ein Kühlmittel (40) einbringbar ist.

5. Verfahren zum Crimpen eines Implantats (20), insbesondere einer intraluminalen Endoprothese, welches wenigstens über einen Teil seiner Länge entweder einen komprimierten Zustand oder einen expandierten Zustand einnehmen kann, vorzugsweise unter Anwendung einer Vorrichtung (10, 10') zum Crimpen nach einem der vorhergehenden Ansprüche, mit den folgenden Schritten:
- Einlegen des zunächst im expandierten Zustand vorliegenden Implantats (20) zumindest mit einem Abschnitt in das Innenvolumen (14, 14') eines hohlzylinder- und/oder hohlkegelförmigen, vorzugsweise geflochtenen Drahtnetzes und
- Verkleinerung des Innendurchmessers durch Aufbringen einer Zugkraft an mindestens einem Ende des Drahtnetzes in Längsrichtung und/oder einer Druckkraft in radialer Richtung auf das Drahtnetz derart, dass das eingelegte Implantat (20) wenigstens entlang eines Abschnitts von dem expandierten Zustand in den komprimierten Zustand überführt wird.

6. Verfahren nach Anspruch 5, **dadurch gekennzeichnet, dass** das Implantat (20) und vorzugsweise auch die Vorrichtung (10, 10') zum Crimpen vor Überführung des Implantats in den komprimierten Zustand in einem Kühlmittel (40) unter die Übergangstemperatur abgekühlt werden.

7. Verfahren nach einem der Ansprüche 5 oder 6, **dadurch gekennzeichnet, dass** zum Aufbringen einer Zugkraft ein an einem ersten Ende des Drahtnetzes in Längsrichtung ein dort angebrachter Ring (17) vorzugsweise auf einer Schiene (18) relativ zu dem feststehenden, gegenüber liegenden zweiten Ende des Drahtnetzes verschoben wird.

8. Verfahren nach einem der Ansprüche 5 bis 7, **dadurch gekennzeichnet, dass** nach dem Überführen in den komprimierten Zustand der komprimierte Abschnitt des Implantats in einen rohrförmigen Außenschaft eines Katheters eingeführt wird.

9. Verfahren nach Anspruch 8, **dadurch gekennzeichnet, dass** das Implantat in einem zweiten Abschnitt, der zunächst nicht in den Außenschaft eingeführt wurde, vorzugsweise mittels der Vorrichtung und/oder nach dem in einem in den Ansprüchen 5 bis 7 angegebenen Verfahren ebenfalls in den komprimierten Zustand überführt wird und anschließend in den Außenschaft eingeführt wird.
